# DEMANDE DE BREVET EUROPEEN

(11) **EP 4 450 052 A1**
(43) Date de publication de la demande: **23.10.2024**
(21) Numéro de dépôt: 23305594.6
(22) Date de dépôt: 18.04.2023
(51) Int. Cl.: A61K 8/365, A61K 8/73, A61K 8/9728, A61Q 1/02, A61Q 19/00, A61K 8/49, A61K 8/36, A61K 8/37, A61K 8/44, A61K 8/46

(54) **COMPOSITION À BASE DE CHITOSAN ET D'UN COMPOSÉ SPÉCIFIQUE**

(71) Demandeur: L'OREAL, 75008 Paris (FR)
(72) Inventeur: LE VERGE, Danielle, 94550 Chevilly Larue (FR); DAVID, Bernadette, 94550 Chevilly Larue (FR); KUSINA, Christophe, 94550 Chevilly Larue (FR)
(74) Mandataire: Lavoix

(57) **Abrégé**

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids ; et
b) au moins un composé choisi parmi les tensioactifs anioniques de type carboxylate, phosphate, sulfonate, sulfate et/ou leurs mélanges.

## Description

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un composé choisi parmi les tensioactifs anioniques de type carboxylate, phosphate, sulfonate, sulfate et/ou leurs mélanges.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes. Par ailleurs, les agents filmogènes couramment utilisés sont généralement de nature pétrochimique, comme les silicones ou les polymères acryliques.

Par ailleurs, on cherche souvent à obtenir des compositions couvrantes.

Les systèmes à base de chitosan montrent que l'association de chitosan et d'un pigment permet de générer des films résistants à la friction à sec et à l'huile. Néanmoins, la sensibilité à l'eau de ces dépôts est très forte et reste un challenge majeur des technologies à base de chitosan.

Le formulateur est donc à la recherche de matières premières et/ou de systèmes permettant d'obtenir des compositions fluides à base de chitosan (i.e. qui s'écoulent) dont le dépôt est couvrant, homogène, présentant une bonne tenue et dont la résistance à l'eau est améliorée.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques aqueuses, colorées ou non, présentant de bonnes propriétés, notamment en termes d'homogénéité et de bonne tenue, en particulier ayant une bonne tenue/résistance à l'eau.

Après application, ces compositions laissent un dépôt filmogène couvrant et homogène, qui a une bonne tenue à l'usure. Les films colorés formés sont adhésifs et cohésifs, et présentent une résistance améliorée à l'eau.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier de la peau, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un composé choisi parmi les tensioactifs anioniques de type carboxylate, phosphate, sulfonate, sulfate et/ou leurs mélanges.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

De préférence, la composition selon l'invention est substantiellement exempte de polymère (méth)acrylique. De préférence, la composition selon l'invention est substantiellement exempte de silicone.

Par « substantiellement exempte de polymère (méth)acrylique », on entend que la composition comprend moins de 1% en poids par rapport au poids total de la composition, de préférence moins de 0,5% en poids, de préférence moins de 0,3% en poids, préférentiellement 0,1% en poids de polymère (méth)acrylique. De préférence, la composition est totalement exempte de polymère (méth)acrylique. Par polymère (méth)acrylique, on entend un polymère comprenant au moins un monomère d'acide acrylique ou au moins un monomère d'acide méthacrylique.

Par « substantiellement exempte de silicone », on entend que la composition comprend moins de 1 % en poids par rapport au poids total de la composition, de préférence moins de 0,5% en poids, de préférence moins de 0,3% en poids, préférentiellement 0,1% en poids de silicone. De préférence, la composition est totalement exempte de silicone. Par silicone, on entend tout composé siliconé.

### Chitosan

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine) -poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

### Tensioactifs anioniques

La composition selon la présente invention comprend un ou plusieurs tensioactifs anioniques.

On entend par tensioactif anionique, un tensioactif ne comportant à titre de groupements ioniques ou ionisables uniquement des groupements anioniques.

Dans la présente description, on qualifie une entité comme étant "anionique" lorsqu'elle possède au moins une charge négative permanente ou lorsqu'elle peut être ionisée en une entité chargée négativement, dans les conditions d'utilisation de la composition de l'invention (milieu, pH par exemple) et ne comprenant pas de charge cationique.

Le ou les tensioactifs anioniques utilisés dans l'invention sont choisis parmi les tensioactifs de type sulfate, sulfonate, carboxylique (ou carboxylate), phosphate et leurs mélanges.

Selon l'invention, le ratio pondéral entre le chitosan et le tensioactif anionique (i.e. ratio pondéral chitosan : tensioactif anionique) est compris entre 0,1 et 10. De préférence, il est compris entre 0,2 et 10, préférentiellement entre 0,3 et 5, et encore plus préférentiellement entre 0,5 et 3.

### Tensioactif carboxylate

La composition selon l'invention peut comprendre un tensioactif anionique de type carboxylate.

Les tensioactifs anioniques de type carboxylate susceptibles d'être utilisés dans la composition de l'invention comportent au moins une fonction carboxylique ou carboxylate (-COOH ou -COO- ).

De préférence, les tensioactifs anioniques de type carboxylate sont choisis parmi les citrates, les acides gras, les N-acyl aminoacides, les lactylates et leurs sels.

Le ou les tensioactifs anioniques de type carboxylate peuvent être présents dans la composition dans une teneur allant de 0,1% à 10%, de préférence de 0,1% à 5%, préférentiellement de 0,1 à 3%, encore plus préférentiellement de 0,45 à 2%, mieux de 0,75% à 1,5%, en poids par rapport au poids total de la composition.

De préférence, la composition selon l'invention comprend au moins un tensioactif anionique de type carboxylate choisi parmi les acides gras et/ou leurs sels.

On entend par « acide gras » un acide carboxylique à chaîne aliphatique comprenant 8 à 30 atomes de carbone, de préférence, de 12 à 24 atomes de carbone, de préférence de 12 à 18 atomes de carbone et encore plus préférentiellement, de 16 à 18 atomes de carbone. L'acide gras peut être liquide ou non liquide.

Par acide gras liquide, on entend un acide gras liquide à température ordinaire (25 °C) et à pression atmosphérique (760 mm de Hg, soit 1,013.10⁵ Pa).

Les acides gras de l'invention peuvent être saturés ou insaturés.

Les acides gras liquides saturés sont éventuellement ramifiés. Ils peuvent éventuellement comprendre dans leur structure au moins un cycle aromatique ou non. De préférence, ils sont acycliques. On peut citer plus particulièrement l'acide isostéarique.

Les acides gras insaturés présentent dans leur structure au moins une double ou triple liaison, et de préférence, une ou plusieurs doubles liaisons. Lorsque plusieurs doubles liaisons sont présentes, elles sont de préférence au nombre de 2 ou 3 et elles peuvent être ou non conjuguées. Ces acides gras insaturés peuvent être linéaires ou ramifiés. Ils peuvent éventuellement comprendre dans leur structure au moins un cycle aromatique ou non. De préférence, ils sont acycliques.

Les acides gras convenant à la mise en oeuvre de l'invention sont plus particulièrement choisis parmi les acides saturés ou insaturés, linéaires ou ramifiés, comportant de 8 à 30 atomes de carbone.

De préférence, l'acide gras est choisi parmi l'acide stéarique, l'acide palmitique, l'acide palmitoléique, l'acide linoléique, l'acide caprylique, l'acide myristique, l'acide laurique, l'acide béhénique, et l'acide isostéarique.

A titre d'exemples d'acides gras, on peut citer l'acide palmitique commercialisé par Cayman Chemical, l'acide palmitique disponible sous la dénomination commerciale EDENOR C16-98 MY, l'acide oléique disponible sous la dénomination commerciale EDENOR OL 75 MY, l'acide isostéarique disponible sous la dénomination commerciale PRISORINE 3505-LQ-(GD), l'acide béhénique disponible sous la dénomination commerciale PALMERA A8522 .

L'acide gras peut être sous forme d'acide libre ou sous forme partiellement ou totalement salifiée. Lorsqu'il est sous forme salifiée, il s'agit notamment de sels alcalins ou d'amines tels que les sels de sodium, de potassium ou de lithium, de sels alcalino-terreux tels que les sels de calcium, de magnésium ou de strontium ; de sel d'aluminium, de zirconium ou de zinc ; de sel d'ammonium, de sel d'un amino-alcool comme le sel de (2-hydroxy éthyl) de sel de diéthanolamine, de sel de triéthanolamine ou encore de sel de triisopropanolamine. De préférence, les sels sont des sels d'aluminium, de magnésium, de calcium, de zirconium, de zinc, de sodium ou de potassium.

A titre d'exemples, on peut notamment citer le caprate de sodium ou de potassium, le caprylate de sodium ou de potassium, le laurate de sodium ou potassium ou de magnésium ou de calcium ou d'ammonium ou de triéthanolamine, le myristate de sodium ou de potassium ou de magnésium ou de calcium ou de diéthanolamine ou de triéthanolamine ou de triisopropanolamine, le palmitate de sodium ou de potassium ou de magnésium ou de triéthanolamine, le cocoate de sodium ou de potassium ou de magnésium ou de triéthanolamine, le stéarate de sodium ou de potassium ou de magnésium ou de calcium ou d'ammonium ou de diéthanolamine ou de triéthanolamine, l'oléate de sodium ou de potassium ou d'ammonium, l'arachidate de sodium ou le béhénate sodium ou de potassium ou de calcium.

Selon un autre mode de réalisation, la composition selon l'invention comprend un tensioactif anionique de type carboxylate choisi parmi les N-acyl amino acides et leurs sels.

Le N-acyl amino acide est un acide aminé présentant un groupement acyle sur l'azote de l'acide aminé.

L'acide aminé peut être, par exemple, la lysine, la glycine, l'acide glutamique ou l'alanine. De préférence, l'acide aminé est choisi parmi la glycine ou l'acide glutamique, plus préférentiellement l'acide aminé est l'acide glutamique.

Le N-acyl amino acide peut être sous forme d'acide libre ou sous forme partiellement ou totalement salifiée. Lorsqu'il est sous forme salifiée, il s'agit notamment de sels alcalins tels que les sels de sodium, de potassium ou de lithium, de sels alcalino-terreux tels que les sels de calcium, de magnésium ou de strontium ; de sel d'aluminium, de zirconium ou de zinc ; de sel d'ammonium ou de sel d'un amino-alcool comme le sel de (2-hydroxy éthyl) ammonium. De préférence, les sels sont des sels d'aluminium, de magnésium, de calcium, de zirconium, de zinc, de sodium ou de potassium.

Le N-acyl amino acide peut comprendre un groupe acyle ayant de 8 à 22 atomes de carbone, tel que, par exemple, un groupe 2-éthylhexanoyle, caproyle, lauroyle, myristoyle, palmitoyle, stéaroyle ou cocoyle. De préférence, le groupe acyle est un groupe stéaroyle.

A titre d'exemple, on peut citer le stéaroyl glutamate de sodium commercialisé sous la dénomination AMISOFT HS 11PF ou le stéaroyl glutamate de disodium commercialisé sous la dénomination AMISOFT HS 21P.

De préférence, le tensioactif anionique de type carboxylate est un dérivé d'acide glutamique et/ou un de ses sels, et plus particulièrement un stéaroyl glutamate, par exemple le stéaroyl glutamate de disodium, le stéaroyl glutamate de sodium ou le stéaroyl glutamate d'aluminium.

De préférence, le tensioactif anionique est le stéaroyl glutamate de disodium.

Selon un autre mode de réalisation particulier, la composition selon l'invention comprend tensioactif anionique de type carboxylate qui est un dérivé de la glycine et/ou un de ses sels.

De préférence, ledit tensioactif anionique glycinate est un acylglycinate en C6-C24, notamment en C10 -C20, plus préférentiellement un cocoylglycinate, encore plus préférentiellement un sel de métal alcalin de cocoylglycinate, et encore plus préférentiellement le sodium cocoyl glycinate.

Par exemple, le sodium cocoyl glycinate est commercialisé sous le nom Amilite^{®} GCS par la société Ajinomoto.

Selon un autre mode de réalisation, le tensioactif anionique de type carboxylate est choisi parmi les citrates.

De préférence, le tensioactif anionique de type carboxylate est le glycéryl stéarate citrate disponible sous la dénomination commerciale AXOL C 62 PELLETS.

Le ou les acides gras, les N-acyl amino acides, les citrates et/ou leurs sels peuvent être présents dans la composition dans une teneur allant de 0,1% à 10%, de préférence de 0,1% à 5%, préférentiellement de 0,1 à 3%, encore plus préférentiellement de 0,45 à 2%, mieux de 0,75% à 1,5%, en poids par rapport au poids total de la composition.

Selon l'invention, le ratio pondéral entre le chitosan et le tensioactif anionique de type carboxylate (i.e. ratio pondéral chitosan : tensioactif anionique) est compris entre 1 et 10. De préférence, il est compris entre 1 et 5, préférentiellement entre 1 et 3, et encore plus préférentiellement entre 1 et 2.

### Tensioactif sulfonate

La composition selon l'invention peut comprendre un tensioactif anionique de type sulfonate.

Les tensioactifs anioniques de type sulfonate susceptibles d'être utilisés dans la composition de l'invention comportent au moins une fonction sulfonate (-SO3H ou -SO3⁻). Ils peuvent être choisis parmi les composés suivants : les alkylsulfonates, les alkylamidesulfonates, les alkylarylsulfonates, les alpha-oléfine-sulfonates, les paraffine-sulfonates, les alkylsulfosuccinates, les alkyléthersulfosuccinates, les alkylamidesulfosuccinates, les alkylsulfoacétates, les N-acyltaurates, les acyliséthionates ; les alkylsulfolaurates ; ainsi que les sels de ces composés; les groupes alkyle de ces composés comportant de 6 à 30 atomes de carbone, notamment de 12 à 28, encore mieux de 14 à 24, voire de 16 à 22, atomes de carbone ; le groupe aryle désignant de préférence un groupe phényle ou benzyle ;ces composés pouvant être polyoxyalkylénés, notamment polyoxyéthylénés et comportant alors de préférence de 1 à 50 motifs oxyde d'éthylène, mieux de 2 à 10 motifs oxyde d'éthylène.

De préférence, le tensioactif est un tensioactif anionique est un tensioactif anionique sulfosuccinate choisi parmi les alkylsulfosuccinates, les alkyléthersulfosuccinates, les alkylamidesulfosuccinates ; de préférence il est un alkylsulfosuccinate.

Préférentiellement, ledit tensioactif anionique sulfosuccinate est un alkylsulfosuccinate en C6-C24, encore plus préférentiellement un laurylsulfosuccinate, encore plus préférentiellement un sel de métal alcalin de laurylsulfosuccinate et encore plus préférentiellement le disodium lauryl sulfosuccinate.

Par exemple, le disodium lauryl sulfosuccinate est commercialisé sous le nom Rewopol^{®} SB 12 P par la société Evonik.

Une composition selon l'invention comprend en outre un tensioactif anionique iséthionate. De préférence, ledit tensioactif anionique iséthionate est un acyliséthionate en C6-C24, notamment en C10-C20, plus préférentiellement un cocoyl iséthionate, encore plus préférentiellement un sel de métal alcalin de cocoyl iséthionate et encore plus préférentiellement le sodium cocoyl iséthionate.

Par exemple, le sodium cocoyl iséthionate est commercialisé sous le nom Hostapon^{®} SCI par la société Clariant ou sous le nom Jordapon^{®} CI par la société BASF.

Lorsque le ou les tensioactifs anioniques de type sulfonate sont sous forme de sel, ledit sel peut être choisi parmi les sels de métaux alcalins, tels que le sel de sodium ou de potassium, les sels d'ammonium, les sels d'amines, et en particulier d'aminoalcools, les sels de métaux alcalino-terreux, tel que le sel de magnésium, et leurs mélanges.

Le ou les tensioactifs anioniques de type sulfonate peuvent être présents dans la composition dans une teneur allant de 0,1% à 10%, de préférence de 0,1% à 5%, préférentiellement de 0,1 à 3%, encore plus préférentiellement de 0,45 à 2%, mieux de 0,75% à 1,5%, en poids par rapport au poids total de la composition.

Selon l'invention, le ratio pondéral entre le chitosan et le tensioactif anionique de type sulfonate (i.e. ratio pondéral chitosan : tensioactif anionique) est compris entre 1 et 10. De préférence, il est compris entre 1 et 5, préférentiellement entre 1 et 3, et encore plus préférentiellement entre 1 et 2.

### Tensioactif sulfate

La composition selon l'invention peut comprendre un tensioactif anionique de type sulfate. Les tensioactifs anioniques de type sulfate susceptibles d'être utilisés dans la composition de l'invention comporte une ou plusieurs fonctions sulfates (-OSO3H ou -OSO3⁻).

De tels tensioactifs peuvent avantageusement être choisis parmi les alkylsulfates, les alkyléthersulfates, les alkylamidosulfates, les alkylamidoéthersulfates, les alkylarylpolyéthersulfates, les monoglycéride-sulfates ; ainsi que leurs sels et leurs mélanges ; les groupes alkyles de ces composés comportant notamment de 6 à 30 atomes de carbone, de préférence de 8 à 26, et plus préférentiellement de 10 à 22 atomes de carbone.

De préférence, le ou les tensioactifs anioniques de type sulfate sont choisi parmi les alkylsulfates notamment en C8 à C26, et de préférence en C10 à C22.

Parmi les tensioactifs anioniques de type sulfates, on peut notamment citer le sodium coco sulfate, le sulfate de lauryl de sodium ou le laureth sulfate de sodium.

De préférence, le composé choisi parmi les tensioactifs anioniques est le sodium coco sulfate commercialisée sous la dénomination RHODAPON CAS 100N MB.

Lorsque le ou les tensioactifs anioniques de type sulfate sont sous forme de sel, ledit sel peut être choisi parmi les sels de métaux alcalins, tels que le sel de sodium ou de potassium, les sels d'ammonium, les sels d'amines, et en particulier d'aminoalcools, les sels de métaux alcalino-terreux, tel que le sel de magnésium, et leurs mélanges.

Le ou les tensioactifs anioniques de type sulfate peuvent être présents dans la composition dans une teneur allant de 0,1% à 10%, de préférence de 0,1% à 5%, préférentiellement de 0,1 à 3%, encore plus préférentiellement de 0,45 à 2%, mieux de 0,75% à 1,5%, en poids par rapport au poids total de la composition.

Selon l'invention, le ratio pondéral entre le chitosan et le tensioactif anionique de type sulfate (i.e. ratio pondéral chitosan : tensioactif anionique) est compris entre 1 et 10. De préférence, il est compris entre 1 et 5, préférentiellement entre 1 et 3, et encore plus préférentiellement entre 1 et 2.

### Matières colorantes pigmentaires

La composition selon l'invention peut comprendre en outre au moins une matière colorante pigmentaire. Cette matière colorante est choisie parmi les matières colorantes pulvérulentes comme les pigments minéraux, les nacres, les pigments organiques.

On entend par « pigments » des particules blanches ou colorées, minérales ou organiques, insolubles dans un milieu aqueux, destinées à colorer la composition et/ou le dépôt résultant.

Les matières colorantes peuvent être présentes, dans la composition, en une teneur allant de 1% à 70%en poids par rapport au poids total de la composition, de préférence de 1% à 60%, de préférence de 3% à 50%, de préférence de 3% à 45% en poids, par rapport au poids de la composition, de préférence de 4% à 30% en poids, de préférence de 5% à 20% en poids, de préférence de 6% à 15% en poids.

### Pigments minéraux

Selon un mode de réalisation particulier, les pigments utilisés selon l'invention sont choisis parmi les pigments minéraux.

Par « pigment minéral », on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : Ta2O5, Ti3O5, Ti2O3, TiO, ZrO2 en mélange avec TiO2, ZrO2, Nb2O5, CeO2, ZnS.

La taille du pigment utile dans le cadre de la présente invention est en général supérieure à 100 nm et peut aller jusqu'à 10 µm, de préférence de 200 nm à 5 µm, et plus préférentiellement de 300 nm à 1 µm. Selon une forme particulière de l'invention, les pigments présentent une taille caractérisée par un D[50] supérieur à 100 nm et pouvant aller jusqu'à 10 µm, de préférence de 200 nm à 5µm, et plus préférentiellement de 300 nm à 1 µm. Les tailles sont mesurées par diffusion statique de la lumière au moyen d'un granulomètre commercial de type MasterSizer 3000^{®} de chez Malvern, permettant d'appréhender la répartition granulométrique de l'ensemble des particules sur une large gamme pouvant aller de 0,01 µm à 1000 µm. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957. D[50] représente la taille maximale que présente 50 % en volume les particules.

Dans le cadre de la présente invention, les pigments minéraux sont plus particulièrement l'oxyde de fer et/ou le dioxyde de titane.

Comme pigments minéraux utilisables dans l'invention, on peut également citer les nacres. Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température. Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Selon un mode particulier, la composition selon l'invention comprend au moins un pigment non enrobé.

### Pigments organiques

Selon un autre mode de réalisation de l'invention, la matière colorante pigmentaire est un pigment organique, synthétique, naturel ou d'origine naturelle.

Par « pigment organique », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Les pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque.

Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090). A titre d'exemples de laques, on peut citer le produit connu sous la dénomination D&C Red 7 (CI 15 850 :1).

### Charges

La composition selon l'invention peut comprendre également au moins une charge.

Par « charges », il faut comprendre des particules de toute forme, incolores ou blanches, minérales ou de synthèses, insolubles et dispersées dans le milieu de la composition quelle que soit la température à laquelle la composition est fabriquée. De manière générale, les charges comprises dans les compositions selon l'invention ne sont pas des matières colorantes pigmentaires.

De préférence, la charge est choisie parmi les particules de cellulose, les silices, l'amidon, le kaolin, les argiles, les particules de nylon ou de polyméthacrylate de méthyle (PMMA) et leurs mélanges.

Les particules de cellulose utilisables selon l'invention sont de préférence sphériques (billes de cellulose).

Par particules sphériques au sens de la présente invention, on entend des particules pleines ou poreuses ayant un paramètre de circularité d'au moins 0,95. Le paramètre de circularité est défini comme le rapport de la circonférence d'un disque ayant la même aire que la particule au périmètre de la particule. Une valeur de 1 caractérise des particules parfaitement sphériques.

Elles présentent de préférence une taille moyenne inférieure à 40 µm, de préférence allant de 1 à 20 µm, encore préférentiellement de 2 à 10 µm.

Parmi les particules de cellulose utilisables selon l'invention, on peut citer en particulier celles vendues par la société Daito sous la marque CELLULOBEADS^{®} telles que CELLULOBEADS USE^{®} (D[50] = 4 µm), CELLULOBEADS D-5^{®} (D[50] < 10 µm), CELLULOBEADS D-10^{®} (D[50] < 15 µm), CELLULOBEADS D-30^{®} (D[50] < 30 µm).

De préférence, les charges sont présentes dans une teneur allant de 0,1% à 20% en poids par rapport au poids total de la composition, de préférence de 0,2% à 15% en poids, par rapport au poids de la composition, de préférence de 0,3% à 10% en poids, de préférence de 0,4% à 5% en poids, de préférence de 0,5% à 4% en poids.

### Alpha hydroxy acide (AHA)

La composition selon l'invention peut comprendre au moins un AHA.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les α-hydroxyacides (alpha hydroxyacides ou AHA) sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide citrique, l'acide malique, l'acide tartrique et leurs sels. Plus particulièrement, l'alpha hydroxyacide est choisi parmi l'acide lactique, l'acide citrique, leurs sels et leurs mélanges.

Le ou les alpha hydroxyacides peuvent être présents en une quantité allant de 0,001 à 10% en poids, de 0,005 à 5% en poids, de préférence de 0,01 à 3% en poids par rapport au poids total de la composition.

### Milieu aqueux physiologiquement acceptable

La composition selon l'invention comprend un milieu aqueux physiologiquement acceptable. Ledit milieu comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en C₁-C₁₀, plus préférentiellement en C₁-C₅, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol, le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 1% à 25% en poids de solvant organique miscible dans l'eau, par rapport au poids total de la composition, plus préférentiellement de 2% à 20% en poids, encore plus préférentiellement de 3% à 15% en poids.

### pH de la composition

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide sont notamment utilisées pour ajuster le pH final de la composition entre 3 et 6,3.

L'acide peut par exemple être l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

### Exemples: préparation d'une composition selon l'invention et comparaisons avec des compositions comparatives

Les compositions B-J selon l'invention, et A comparative, ont été préparées par mélange des ingrédients du tableau 1.

Les mélanges ont été faits en chauffant à 60°C pendant l'étape de mélange pour permettre l'homogénéité.

### Protocole d'étalement des compositions en un film

L'étalement des produits se fait sur un banc d'étalement (Elcometer 4340 Applicator) permettant de régler sa vitesse ainsi que la distance sur lequel il se fait. Le banc est équipé d'un système d'aspiration relié à une pompe pour que le support où l'on fait l'étalement, ne bouge pas. Des cartes de contraste avec un fond noir et un fond blanc non vernis sont utilisées (1 byko-chart, uncoated N2A, code 2831). L'épaisseur d'étalement est quant à elle réglable grâce à l'étaleur carré déposé sur le support de manière à étaler par arasement lorsque la plateforme est mise en marche. Chaque tranche de l'étaleur permet d'étaler avec une épaisseur différente allant de 25µm à 200µm. L'épaisseur choisie est de 25 µm afin de se rapprocher d'une épaisseur du film *in vivo.* Un poids de 960 g est ajouté par-dessus l'étaleur pendant l'étalement. La vitesse de l'étalement est réglée à 1in/sec, soit 2,54cm/s. Les films sont séchés pendant 24h à température et humidité (HR) ambiante (50% HR).

### Protocole de test de tenue aux frottements

Le test de tenue aux frottements à l'eau est réalisé par les mesures colorimétriques sur film sec avant et après abrasion. L'abrasion est effectuée en fixant une bandelette de mouchoir en tissu (Chicopee Veraclean Polish Plus) mouillé (400 µL d'eau dans le mouchoir en tissu) sur la tranche d'étaleur à 25 µm. Le poids de 960 g est ajouté par-dessus l'étaleur pendant l'abrasion. La vitesse de banc est réglée à 2,54 cm/s.

La mesure de la couleur avant est après abrasion est faite avec la même méthode spectrophotométrique qu'expliquée précédemment.

Afin d'évaluer la tenue aux frottements, le « Contraste Ratio » respectivement avant le frottement (CR Dépôt Sec, %) et après l'abrasion (CR Frot Dépôt Sec, %) sont mesurés. La perte [(CR Frot Dépôt Sec - CR Dépôt sec] / CR Dépôt Sec]*100, en pourcentage, quantifie la perte de couvrance et indique la tenue du film aux frottements : plus cette perte est faible, plus le film est résistant aux frottements.

Chaque valeur de CR représente donc une moyenne de 3 à 6 mesures. Les résultats sont dans le tableau 1.

**[Table 1]**

| **Ingrédients (% p/p)** | **A compar ative** | **B selon l'inventi on** | **C selon l'inventi on** | **D selon l'inventi on** | **E selon l'inventi on** | **F selon l'inventi on** | **G selon l'inventio n** | **H selon l'inventio n** | **I selon l'inventio n** | **J selon l'inventio n** |
|---|---|---|---|---|---|---|---|---|---|---|
| Chitosan (Kiosmetine -CSH de Kitozyme) | 2 | 2 | 2 | 2 | 2 | 2 | 2 | 2 | 2 | 2 |
| Acide lactique | 1 | 1 | 1 | 1 | 1 | 1 | 1 | 1 | 1 | 1 |
| Pigments* | 15 | 15 | 15 | 15 | 15 | 15 | 15 | 15 | 15 | 15 |
| Stearoyl de glutamate de disodium (AMISOFT HS 21 P) | | 1 | | | | | | | | |
| Lauroyl de glutamate de sodium (AMISOFT LS 11) | | | 1 | | | | | | | |
| Stearoyl de glutamate de sodium (AMISOFT HS 11 PF) | | | | 1 | | | | | | |
| Acide oléique (EDENOR OL 75 MY) | | | | | 1 | | | | | |
| Acide isostéarique (PRISORINE 3505-LQ-(GD)) | | | | | | 1 | | | | |
| Acide béhénique (PALMERA A8522) | | | | | | | 1 | | | |
| Acide palmitique (EDENOR C16-98 MY) | | | | | | | | 1 | | |
| Glycéryl de stéarate citrate (AXOL C 62 PELLETS MB) | | | | | | | | | 1 | |
| Coco sulfate de sodium (RHODAPO N CAS 100N MB) | | | | | | | | | | 1 |
| Eau | Qsp 100 | Qsp 100 | Qsp 100 | Qsp 100 | Qsp 100 | Qsp 100 | Qsp 100 | Qsp 100 | Qsp 100 | Qsp 100 |
| **Perte friction eau % (+/-%)** | -54 (13) | -17(2) | -29 (8) | -23 (6) | -18 (4) | 2(1) | -23 (4) | -19(4) | -21 (4) | -19(4) |

| | | | | | | | | | | |
|---|---|---|---|---|---|---|---|---|---|---|
| * Les pigments utilisés dans les compositions sont un mélange de dioxyde de titane commercialisé sous la dénomination HOMBITAN FF PHARMA-VENATOR, d'oxyde de fer rouge commercialisé sous la dénomination TAROX IRON OXIDE R-800HP - TITAN KOGYO, d'oxyde de fer jaune commercialisé sous la dénomination TAROX IRON OXIDE BL-100HPL- TITAN KOGYO et d'oxyde de fer noir commercialisé sous la dénomination TAROX IRON OXIDE LL-100HP- TITAN KOGYO. | | | | | | | | | | |

Les résultats montrent une meilleure tenue à l'eau pour les compositions B, C, D, E, F, G, H, I et J selon l'invention, par rapport à la composition comparative A. L'utilisation d'un tensioactif anionique utilisé dans l'invention, apporte ainsi de la résistance à l'eau.

### Influence de la teneur en tensioactif anionique sur la résistance à l'eau

Les compositions K, L, M et N selon l'invention, et O comparative, ont été préparées par mélange des ingrédients du tableau 2.

Les compositions K, L, M et N selon l'invention contiennent des pigments, et du stéaroyl de glutamate disodique dans une teneur allant de 0,45% à 1,5% en poids par rapport au poids total de la composition.

La composition O comparative contient du chitosan ainsi que des pigments, mais pas tensioactifs anioniques.

Puis la tenue à l'eau est évaluée. Le protocole d'évaluation est le suivant :
- Appliquer chaque composition sur une zone de chaque avant-bras (avec une zone d'avant-bras supplémentaire = témoin) : pour cela, on dépose 10µl de chaque composition sur une zone d'avant-bras de surface 2,5 x 2,5 cm pendant 15 s,
- Laisser sécher 20 min,
- Déposer 100 µL d'eau sur un tissu de frottement en coton,
- Appliquer le coton sur la zone traitée à l'aide d'un poids de 900 g pendant 5 s,
- Retirer le coton avec une force comprise entre 100 et 300 g, et
- Evaluer visuellement le dépôt témoin versus le dépôt frotté à l'eau. Cette évaluation comprend un scorage de tenue à l'eau, qui va de (-) = aucune tenue à l'eau, à (+++++) = excellente tenue à l'eau.

**[Table 2]**

| **Ingrédients (% p/p)** | **O compar ative** | **K selon l'inventi on** | **L selon l'inventi on** | **M selon l'inventi on** | **N selon l'inventi on** |
|---|---|---|---|---|---|
| Chitosan (Kiosmetine-CSH de Kitozyme) | 2 | 2 | 2 | 2 | 2 |
| Acide lactique | 1 | 1 | 1 | 1 | 1 |
| Pigments* | 15 | 15 | 15 | 15 | 15 |
| Stearoyl de glutamate disodique (AMISOFT HS 21P) | | 0,45 | 0,75 | 1 | 1,5 |
| Eau | Qsp 100 | Qsp 100 | Qsp 100 | Qsp 100 | Qsp 100 |
| **Note tenue à l'eau (de 1 (-) à 5 (+))** | 1 | 3 | 4 | 4 | 5 |

| | | | | | |
|---|---|---|---|---|---|
| * Les pigments utilisés dans les compositions sont un mélange de dioxyde de titane commercialisé sous la dénomination HOMBITAN FF PHARMA-VENATOR, d'oxyde de fer rouge commercialisé sous la dénomination TAROX IRON OXIDE R-800HP - TITAN KOGYO, d'oxyde de fer jaune commercialisé sous la dénomination TAROX IRON OXIDE BL-100HPL- TITAN KOGYO et d'oxyde de fer noir commercialisé sous la dénomination TAROX IRON OXIDE LL-100HP- TITAN KOGYO. | | | | | |

Les résultats montrent une meilleure tenue à l'eau pour les compositions K, L, M et N selon l'invention, par rapport à la composition comparative O. On observe une amélioration de la tenue à l'eau lorsque la quantité de tensioactif anionique est de 1,5%. L'utilisation d'une quantité supérieure (1,5%) de tensioactif anionique dans la composition selon l'invention, apporte ainsi une meilleure résistance à l'eau.

## Revendications

1. Composition cosmétique, notamment de maquillage et/ou de soin de la peau et/ou des lèvres, en particulier de la peau, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un composé choisi parmi les tensioactifs anioniques de type carboxylate, phosphate, sulfonate, sulfate et/ou leurs mélanges.

2. Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

3. Composition selon la revendication 1 ou 2, dans laquelle le chitosan natif a un degré d'acétylation du chitosan inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

4. Composition selon l'une des revendications précédentes, dans laquelle le chitosan natif est un polysaccharide préparé à partir d'une origine fongique, de préférence issu du mycélium d'un champignon du type Ascomycète, et en particulier *d'Aspergillus niger* et/ou d'un champignon Basidiomycète, et en particulier *Lentinula edodes* et/ou *Agaricus bisporus,* de préférence le champignon est *Aspergillus niger.*

5. Composition selon l'une des revendications précédentes, dans laquelle le chitosan natif est présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

6. Composition selon l'une des revendications précédentes, qui comprend au moins un composé choisi parmi les matières colorantes pigmentaires et les charges.

7. Composition selon la revendication 6, dans laquelle la matière colorante pigmentaire est un pigment minéral choisi parmi le dioxyde de titane, les oxydes de fer, les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre, les nacres, les pigments monochromatiques ; et/ou la charge est choisie parmi les particules de cellulose, les silices, l'amidon, le kaolin, les argiles, les particules de nylon ou de polyméthacrylate de méthyle et leurs mélanges.

8. Composition selon la revendication 6, dans laquelle la matière colorante pigmentaire est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

9. Composition selon la revendication 6 ou 7, dans laquelle la matière colorante pigmentaire est choisie parmi les pigments minéraux, de préférence parmi le dioxyde de titane, les oxydes de fer, les oxydes de zirconium ou de cérium, les oxydes de zinc ou de chrome et leurs mélanges, de préférence le pigment est choisi parmi le dioxyde de titane, les oxydes de fer et leurs mélanges.

10. Composition selon l'une des revendications 6 à 9, dans laquelle la matière colorante pigmentaire est présente en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

11. Composition selon l'une des revendications précédentes, dans laquelle le composé choisi parmi les tensioactifs anioniques est un tensioactif anionique de type carboxylates choisi parmi les citrates, les acides gras, les N-acyl amino acides, les lactylates et/ou leurs sels.

12. Composition selon l'une des revendications précédentes, dans laquelle le tensioactif anionique de type carboxylate est choisi parmi les acides gras et/ou leurs sels, notamment parmi l'acide stéarique, l'acide palmitique, l'acide palmitoléique, l'acide linoléique, l'acide caprylique, l'acide myristique, l'acide laurique, l'acide béhénique et l'acide isostéarique.

13. Composition selon l'une des revendications précédentes, dans laquelle le tensioactif anionique est un N-acyl amino acide comprenant un groupe acyle ayant de 8 à 22 atomes de carbone, tel qu'un groupe 2-éthylhexanoyle, caproyle, lauroyle, myristoyle, palmitoyle, stéaroyle ou cocoyle, de préférence le groupe acyle est un groupe stéaroyle.

14. Composition selon l'une des revendications précédentes, dans laquelle le tensioactif anionique est le stéaroyl glutamate de disodium.

15. Composition selon l'une des revendications précédentes, dans laquelle le tensioactif anionique est choisi parmi les alkylsulfosuccinates, les alkyléthersulfosuccinates et les alkylamidesulfosuccinates, de préférence il est un alkylsulfosuccinate.

16. Composition selon l'une des revendications précédentes, dans laquelle le tensioactif anionique choisi parmi les citrates, les acides gras, les N-acyl amino acides, les lactylates et/ou leurs sels est présent dans la composition dans une teneur allant de 0,1% à 10%, de préférence de 0,1% à 5%, préférentiellement de 0,1 à 3%, encore plus préférentiellement de 0,45 à 2%, mieux de 0,75% à 1,5%, en poids par rapport au poids total de la composition.

17. Composition selon l'une des revendications précédentes, dans laquelle le milieu aqueux physiologiquement acceptable comprend au moins 5% en poids d'eau par rapport au poids total de la composition, de préférence au moins 10% en poids, de préférence au moins 20% en poids, de préférence au moins 30% en poids, de préférence au moins 40% en poids ; de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50% ; et éventuellement au moins un solvant organique miscible dans l'eau à 25°C choisi parmi les alcools, les polyols et leurs mélanges.

18. Composition selon l'une des revendications précédentes, dans laquelle le ratio pondéral entre le chitosan et le tensioactif anionique (i.e. ratio pondéral chitosan : tensioactif anionique) est compris entre 0,1 et 10, de préférence entre 0,2 et 10, préférentiellement entre 0,3 et 5, et encore plus préférentiellement entre 0,5 et 3.

19. Composition selon l'une des revendications précédentes, qui comprend au moins un alpha hydroxy acide, de préférence choisi parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

20. Composition selon l'une des revendications précédentes qui est substantiellement exempte de polymère (méth)acrylique et/ou de silicone ; de préférence la composition comprend moins de 1% en poids par rapport au poids total de la composition, de préférence moins de 0,5% en poids, de préférence moins de 0,3% en poids, préférentiellement 0,1% en poids de polymère (méth)acrylique, de préférence la composition est totalement exempte de polymère (méth)acrylique ; de préférence la composition comprend moins de 1% en poids par rapport au poids total de la composition, de préférence moins de 0,5% en poids, de préférence moins de 0,3% en poids, préférentiellement 0,1% en poids de silicone, de préférence la composition est totalement exempte de silicone.

21. Composition selon l'une des revendications précédentes, qui présente un pH inférieur ou égal à 7, de préférence inférieur ou égal à 6,5, de préférence inférieur ou égal à 6,3, de préférence compris entre 3 et 6,3, de préférence compris entre 4 et 6,3.

22. Procédé de maquillage et/ou de soin de la peau et/ou des phanères, dans lequel on applique la composition selon l'une des revendications précédentes sur la peau et/ou les phanères.
